# EUROPEAN PATENT SPECIFICATION

(11) **EP 2 132 173 B1**
(45) Date of publication and mention of the grant of the patent: **07.10.2015**
(21) Application number: 08725987.5
(22) Date of filing: 22.02.2008
(51) Int. Cl.: A61K 31/165, A61K 9/00, A61K 9/20, A61K 9/48, C07D 209/42, A61P 19/10

(54) **FORMULATIONS FOR CATHEPSIN K INHIBITORS**
FORMULIERUNGEN FÜR CATHEPSIN K-HEMMER
FORMULATIONS POUR INHIBITEURS DE LA CATHEPSINE K

(30) Priority: 26.02.2007 US 903493 P
(43) Date of publication of application: 16.12.2009
(73) Proprietor: Merck Sharp & Dohme Corp., Rahway, NJ 07065-0907 (US); Merck Canada Inc., Kirkland, QC H9H 3L1 (CA)
(72) Inventor: PARENT, Wayne, Kirkland, Québec H9H 3L1 (CA); AFAGHI, Mahtab, Kirkland, Québec H9H 3L1 (CA); BRESLIN, David, Rahway, New Jersey 07065-0907 (US); GRANGER, Mireille, Kirkland, Québec H9H 3L1 (CA); WANG, Lei, Rahway, New Jersey 07065-0907 (US); ZIMMERMAN, Jeff, Rahway, New Jersey 07065-0907 (US)
(74) Representative: Hussain, Deeba
(86) International application number: PCT/US2008/002399
(87) International publication number: WO 2008/106059

(56) References cited:
- EP-B1- 0 658 348
- WO-A1-2005/019161
- WO-A1-2005/019161
- WO-A1-2007/008496
- WO-A2-2007/046842
- US-A1- 2004 073 025
- US-A1- 2006 251 723
- VALENTINO J STELLA: "High Tech, Low Tech or Right Tech? The Discovery and development of a new Pharmaceutical Excipient, Captisol" YAKUZAIGAKU - JOURNAL OF PHARMACEUTICAL SCIENCE AND TECHNOLOGY, YAKUJI NIPPO-SHA, TOKYO, JP, vol. 60, no. Suppl, 1 April 2000 (2000-04-01), pages 11-14, XP009092501 ISSN: 0372-7629
- BONE HENRY G ET AL: "Odanacatib, a cathepsin-K inhibitor for osteoporosis: a two-year study in postmenopausal women with low bone density.", JOURNAL OF BONE AND MINERAL RESEARCH : THE OFFICIAL JOURNAL OF THE AMERICAN SOCIETY FOR BONE AND MINERAL RESEARCH MAY 2010, vol. 25, no. 5, May 2010 (2010-05), pages 937-947, ISSN: 1523-4681
- STOCH S A ET AL: "Effect of the Cathepsin K Inhibitor Odanacatib on Bone Resorption Biomarkers in Healthy Postmenopausal Women: Two Double-Blind, Randomized, Placebo-Controlled Phase I Studies", CLINICAL PHARMACOLOGY & THERAPEUTICS, vol. 86, no. 2, August 2009 (2009-08), pages 175-182, ISSN: 0009-9236

## Description

### BACKGROUND OF THE INVENTION

This invention relates to formulations of cathepsin K inhibitors.

A variety of cathepsin K inhibitors have been disclosed for the treatment of various disorders related to cathepsin K functioning, including osteoporosis, glucocorticoid induced osteoporosis, Paget's disease, abnormally increased bone turn over, tooth loss, bone fractures, rheumatoid arthritis, osteoarthritis, periprosthetic osteolysis, osteogenesis imperfecta, atherosclerosis, obesity, glaucoma, chronic obstructive pulmonary disease and cancer including metastatic bone disease, hypercalcemia of malignancy, and multiple myeloma. Representative examples of cathepsin K inhibitors include those disclosed in International Publication WO03/075836, which published on September 18, 2003, to Merck & Co., Inc. & Axys Pharmaceuticals.

Cathepsin K inhibitors can be formulated for oral dosing as tablets, by using a direct compression, wet granulation or roller compaction method. Similarly, cathepsin K inhibitors can be formulated for oral dosing as gelatin capsules, being a liquid in a soft capsule, or dry powder or semi-solid in a hard capsule. In addition, cathepsin K inhibitors can be formulated for intravenous dosing.

WO-A-2005019161 (Merck Frosst Canada & Co.) discloses cathepsin K inhibitors, including odanacatib and provides a generic disclosure of tablets and standard ingredients.

### SUMMARY OF THE INVENTION

The instant invention relates to pharmaceutical compositions containing cathepsin K inhibitors. Also disclosed are processes for making said pharmaceutical compositions.

### DETAILED DESCRIPTION OF THE INVENTION

A particularly effective cathepsin K inhibitor is *N*¹-(1-cyanocyclopropyl)-4-fluoro-*N*²-{(1*S*-2,2,2-trifluoro-1-[4'-(methylsulfonyl)-1,1'-biphenyl-4-yl]ethyl}-L-leucinamide, 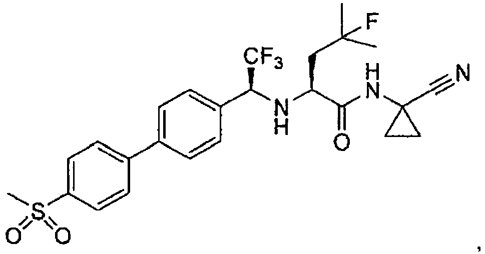 which can be prepared by procedures described in: International Publication WO03/075836, which published on September 18, 2003, to Merck & Co., Inc. & Axys Pharmaceuticals; International Publication WO2006/017455, which published on February 16, 2006, to Merck& Co., Inc.; U.S. Publication US2006-0052642, which published on March 09, 2006; U.S. Publication US2005-0234128, which published on October 20, 2005, to Merck & Co., Inc..

The present invention provides a tablet comprising by weight, 12.5% by weight of *N*¹-(1-cyanocyclopropyl)-4-fluoro-*N*²-{(1*S*)-2,2,2-trifluoro-1-[4'-(methylsulfonyl)-1,1'-biphenyl-4-yl]ethyl}-L-leucinamide, 40% by weight of microcrystalline cellulose, 40% by weight of lactose monohydrate, 4% by weight of croscarmellose sodium, 3% by weight of hydroxypropyl cellulose and 0.5% by weight of magnesium stearate, wherein 50mg of *N*¹-( 1-cyanocyclopropyl)-4-fluoro-*N*²-{(1*S*)-2,2,2-trifluoro-1-[4'-(methylsulfonyl)-1,1'-biphenyl-4-yl]ethyl}-L-leucinamide is present.

Disclosed is a process for the preparation of a tablet containing a cathepsin K inhibitor, which process comprises:
(a) forming a powder blend of the cathepsin K inhibitor with excipients,
(b) wet granulating the powder blend with hydroxypropyl cellulose to form granules,
(c) drying the granules, and
(d) compressing the dried granules in to a tablet.

Also disclosed is a process for the preparation of a tablet containing a cathepsin K inhibitor, which process comprises:
(a) forming a powder blend of the cathepsin K inhibitor with excipients, using a mixer,
(b) wet granulating the powder blend with a binder to form granules,
(c) drying the granules in a fluid bed dryer,
(d) milling the dried granulate,
(e) lubricating the dried granules, and
(f) compressing the dried granules in to a tablet.

Also disclosed is a process for the preparation of a tablet containing a cathepsin K inhibitor, which process comprises:
(a) mixing together the cathepsin K inhibitor, diluents, and a dry binder,
(b) lubricating the mixture from step (a),
(c) dry granulating the lubricated mixture,
(d) size reducing the granules,
(e) lubricating the granules, and
(f) compressing the tablets on a rotary tablet press.

In an embodiment of the process, the cathespin K inhibitor, diluent and dry binder are mixed together in a drum blender for 10 minutes. In a class of the embodiment, the drum blender is set at 46 rpm.

In an embodiment of the process, the mixture from step (a) is lubricated in a drum blender for 1 minute. In a class of the embodiment, the drum blender is set at 46 rpm.

In an embodiment of the process, the lubricated mixture from step (b) is dry granulated on a roller compactor. In a class of the embodiment, the roller compactor is set with a roll pressure of 400 MPa, a roll speed of 4.00 rpm and a screw speed of 55.5 rpm.

In an embodiment of the process, the granules from step (c) are size reduced by milling said granules through a screen and a round rasp screen. In a class of the embodiment, the screen measures 1 mm and the round rasp screen measures 1.27 mm.

The pharmaceutical tablet compositions of the present invention may also contain one or more additional formulation ingredients that may be selected from a wide variety of excipients known in the pharmaceutical formulation art. According to the desired properties of the tablet, any number of ingredients may be selected, alone or in combination, based upon their known uses in preparing tablet compositions. Such ingredients include, but are not limited to, diluents, binders, compression aids, disintegrants, lubricants, flavors, flavor enhancers, sweeteners, preservatives, colorants and coatings.

The term "tablet" as used herein is intended to encompass compressed pharmaceutical dosage formulations of all shapes and sizes, whether uncoated or coated. Substances which may be used for coating include hydroxypropylmethylcellulose, hydroxypropylcellulose, titanium dioxide, talc, sweeteners and colorants.

The pharmaceutical compositions of the present invention are useful in the therapeutic or prophylactic treatment of disorders associated with cathepsin K functioning. Such disorders include: osteoporosis, glucocorticoid induced osteoporosis, Paget's disease, tooth loss, bone fractures, rheumatoid arthritis, osteoarthritis, periprosthetic osteolysis, osteogenesis imperfecta, atherosclerosis, obesity, glaucoma, chronic obstructive pulmonary disease and cancer including metastatic bone disease, hypercalcemia of malignancy, and multiple myeloma.

The following example is given for the purpose of illustrating the present invention and shall not be construed as being a limitation on the scope of the invention.

### Ranges of conditions for processing:

The wet granulation processes disclosed herein can be performed in (but not limited to) high shear mixer and fluid bed processor system. Granule is then milled through a size reduction mill, lubricant is added to the granule contained in a tote, and then mixed. Granule is then compressed into tablets.

The dry granulation process can be performed in (but not limited to) a roller compactor. Granule is then milled through a size reduction mill, lubricant is added to the granule contained in a tote , and then mixed. Granule is then compressed into tablets.

### EXAMPLE 1

### PREPARATION OF 50 MG TABLETS

| Component | % wt./wt. | Mg/Tablet | Weight (kg) (Batch = 100,000 tablet) |
|---|---|---|---|
| *N*¹-(1-cyanocyclopropyl)4-fluoro-*N*²-{(1*S*)-2,2,2-trifluoro-1-[4'-(methylsulfonyl)-1,1'-biphenyl-4-yl]ethyl}-L-leucinamide | 12.5 | 50.00 | 5.0 |
| Microcrystalline Cellulose | 40 | 160.00 | 16.0 |
| Lactose Monohydrate | 40 | 160.000 | 16.0 |
| Croscarmellose Sodium | 4 | 16.00 | 1.6 |
| Hydroxypropyl cellulose | 3 | 12.00 | 1.2 |
| Magnesium Stearate | 0.5 | 2.00 | 0.2 |
| Purified Water* | [35] | [140.00] | [14.0] |
| Total | 100 | 400.00 | 40.0 |

| | | | |
|---|---|---|---|
| * removed during the during process | | | |

*N*¹-(1-cyanocyclopropyl)-fluoro-*N*²-{(1*S*)-2,2,2-trifluoro-1-[4'-(methylsulfonyl)-1,1'-biphenyl-4-yl]ethyl)-L-leucinamide, 4% (wt. /wt.) croscarmellose sodium, and a 1:1 (wt. /wt.) mixture of microcrystalline cellulose and lactose monohydrate are dry blended in a high shear mixer, and then a 3% (wt. /wt.) hydroxypropyl cellulose solution is sprayed onto the mixing powders to effect granulation. The wet granulate is dried in a fluid bed dryer, the dried granulate is then milled, and finally lubricated with 0.5% (wt- /wt.) magnesium stearate in a blender. Tablets were then compressed on a rotary tablet press.

## Claims

1. A tablet comprising by weight, 12.5% by weight of *N*¹-(1-cyanocyclopropyl)-4-fluoro-*N*²-{(1*S*)-2,2,2-trifluoro-1-[4'-(methylsulfonyl)-1,1'-biphenyl-4-yl]ethyl}-L-leucinamide, 40% by weight of microcrystalline cellulose, 40% by weight of lactose monohydrate, 4% by weight of croscarmellose sodium, 3% by weight of hydroxypropyl cellulose and 0.5% by weight of magnesium stearate, wherein 50mg of *N*¹-(1-cyanocyclopropyl)-4-fluoro-*N*²-{(1*S*)-2,2,2-trifluoro-1-[4'-(methylsulfonyl)-1,1'-biphenyl-4-yl]ethyl}-L-leucinamide is present.

2. A tablet of claim 1 for use in the treatment of osteoporosis, glucocorticoid induced osteoporosis, Paget's disease, tooth loss, bone fractures, rheumatoid arthritis, osteoarthritis, periprosthetic osteolysis, osteogenesis imperfect, atherosclerosis, obesity, glaucoma, chronic obstructive pulmonary disease or cancer including metastatic bone disease, hypercalcemia of malignancy, or multiple myeloma.

3. Use of a tablet as defined in claim 1 for the manufacture of a medicament for the treatment of osteoporosis, glucocorticoid induced osteoporosis, Paget's disease, tooth loss, bone fractures, rheumatoid arthritis, osteoarthritis, periprosthetic osteolysis, osteogenesis imperfect, atherosclerosis, obesity, glaucoma, chronic obstructive pulmonary disease or cancer including metastatic bone disease, hypercalcemia of malignancy, or multiple myeloma.

## Patentansprüche

1. Eine Tablette, umfassend, bezogen auf das Gewicht, 12,5 Gew.-% *N*¹-(1-Cyanocyclopropyl)-4-fluor-*N*²-{(1*S*)-2,2,2-trifluor-1-[4'-(methylsulfonyl)-1,1'-biphenyl-4-yl]ethyl}-L-leucinamid, 40 Gew.-% mikrokristalline Cellulose, 40 Gew.-% Lactosemonohydrat, 4 Gew.-% Croscarmellose-Natrium, 3 Gew.-% Hydroxypropylcellulose und 0,5 Gew.-% Magnesiumstearat, wobei 50 mg *N*¹-(1-Cyanocyclopropyl)-4-fluor-*N*²-{(1*S*)-2,2,2-trifluor-1-[4'-(methylsulfonyl)-1,1'-biphenyl-4-yl]ethyl}-L-leucinamid vorliegen.

2. Eine Tablette nach Anspruch 1 zur Verwendung bei der Behandlung von Osteoporose, Glucocorticoid-induzierter Osteoporose, Paget-Krankheit, Zahnausfall, Knochenfrakturen, Gelenkrheumatismus, Arthrose, periprothetischer Osteolyse, Osteogenesis imperfecta, Atherosklerose, Adipositas, Glaukom, chronisch-obstruktiver Lungenerkrankung oder Krebs einschließlich metastatischer Knochenerkrankung, tumorinduzierter Hyperkalzämie oder multiplem Myelom.

3. Verwendung einer Tablette wie in Anspruch 1 definiert zur Herstellung eines Medikaments zur Behandlung von Osteoporose, Glucocorticoid-induzierter Osteoporose, Paget-Krankheit, Zahnausfall, Knochenfrakturen, Gelenkrheumatismus, Arthrose, periprothetischer Osteolyse, Osteogenesis imperfecta, Atherosklerose, Adipositas, Glaukom, chronisch-obstruktiver Lungenerkrankung oder Krebs einschließlich metastatischer Knochenerkrankung, tumorinduzierter Hyperkalzämie oder multiplem Myelom.

## Revendications

1. Comprimé comprenant, en poids, 12,5% en poids de *N*¹-(1-cyanocyclopropyl)-4-fluoro-*N*²- {(1*S*)-2,2,2-trifluoro-1-[4'-(méthylsulfonyl)-1,1'-biphényl-4-yl]éthyl}-L-leucinamide, 40% en poids de cellulose microcristalline, 40% en poids de lactose monohydraté, 4% en poids de croscarmellose sodique, 3% en poids d'hydroxypropyl cellulose et 0,5% en poids de stéarate de magnésium, dans lequel 50mg de *N*¹-(1-cyanocyclopropyl)-4-fluoro-*N*²-{(1*S*)-2,2,2-trifluoro-1-[4'-(méthylsulfonyl)-1,1'-biphényl-4-yl]éthyl}-L-leucinamide sont présents.

2. Comprimé selon la revendication 1 pour l'utilisation dans le traitement d'une ostéoporose, d'une ostéoporose induite par glucocorticoïde, d'une maladie de Paget, d'une perte de dent, de fractures osseuses, d'une polyarthrite rhumatoïde, une ostéoarthrite, d'une ostéolyse périprothétique, d'une ostéogenèse imparfaite, d'une athérosclérose, d'une obésité, d'un glaucome, d'une maladie pulmonaire obstructive chronique ou d'un cancer incluant une maladie osseuse métastatique, une hypercalcémie maligne, ou un myélome multiple.

3. Utilisation d'un comprimé selon la revendication 1 pour la fabrication d'un médicament pour le traitement d'une ostéoporose, d'une ostéoporose induite par glucocorticoïde, d'une maladie de Paget, d'une perte de dent, de fractures osseuses, d'une polyarthrite rhumatoïde, une ostéoarthrite, d'une ostéolyse périprothétique, d'une ostéogenèse imparfaite, d'une athérosclérose, d'une obésité, d'un glaucome, d'une maladie pulmonaire obstructive chronique ou d'un cancer incluant une maladie osseuse métastatique, une hypercalcémie maligne, ou un myélome multiple.
